(19) Europäisches Patentamt
European Patent Office
Office européen des brevets

(11) Veröffentlichungsnummer: **0 081 457**
**B1**

(12) # EUROPÄISCHE PATENTSCHRIFT

(45) Veröffentlichungstag der Patentschrift: **07.10.87**

(21) Anmeldenummer: **82810509.8**

(22) Anmeldetag: **29.11.82**

(51) Int. Cl.[4]: **C 07 C 39/15,** C 07 C 43/178, C 07 C 149/36, C 09 K 15/08, C 09 K 15/14, C 08 L 55/02, C 08 L 25/04, C 08 K 5/13

(54) **Phenole und ihre Verwendung als Stabilisatoren.**

(30) Priorität: **03.12.81 CH 7734/81**

(43) Veröffentlichungstag der Anmeldung: **15.06.83 Patentblatt 83/24**

(45) Bekanntmachung des Hinweises auf die Patenterteilung: **07.10.87 Patentblatt 87/41**

(84) Benannte Vertragsstaaten: **DE FR GB IT**

(56) Entgegenhaltungen:
**EP - A - 0 003 338**
**FR - A - 1 570 349**
**FR - A - 1 576 215**

**Journal of the Soc.of Chemical Industry, Supplem. Issue 1, S3-S7 (1950)**
**Makromolekulare Chemie 9, 1-24 (1952)**
**Mater Plast. Elastomeri 1975, 422-426**
**Chemical Abstract Registry File - P041157E**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Rosenberger, Siegfried, Im Gehracker 11, CH-4125 Riehen (CH)**

**EP 0 081 457 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue metafunktionelle, insbesondere an mindestens 2 aromatischen Kernen OH-Gruppen enthaltende Phenole, welche als Stabilisatoren für organisches Material Verwendung finden.

Es sind bereits einige mindestens an 2 aromatischen Kernen OH-Gruppen enthaltende Phenole bekannt, bei denen die aromatischen Kerne über Brückenradikale verbunden sind und welche die OH-Gruppe jeweils an den Kernen in o- oder p-Stellung zu dem C-Atom enthalten, über das die Verbrückung zum 2. aromatischen Kern erfolgt oder wo die OH-Substitution unbestimmt ist. Auch die Verwendung derartiger Phenole als Stabilisatoren ist bereits bekannt. Derartige Phenole sind beispielsweise in den folgenden Schutzrechtsschriften beschrieben: US 3 346, DE 2 138 839, JP 7 314 181.

Es sind auch bereits wenige mindestens an 2 aromatischen Kernen OH-Gruppen enthaltende Phenole bekannt, welche die OH-Gruppen an den Kernen in m-Stellung zu dem C-Atom enthalten, über das die Verbindung zum 2. aromatischen Kern erfolgt. Auch die Verwendung solcher Phenole als Antioxidantien ist bekannt. Diesbezüglich ist auf DE 2 211 722 und auf eine Publikation von H. Budzikiewicz und J. Swoboda in Chem. Ber. 98 (10) (1965) 3264-9 hinzuweisen.

In der FR-A-1 576 215 wird die stabilisierende Wirkung von bestimmten Bis(hydroxyphenyl) methanderivaten angegeben, die sich als Ausgangsprodukte für polymere Bisphenole eignen.

In einem Artikel von S.R. Finn und J.W.G. Musty in «Journal of the Society of Chemical Industry, Supplementary Issue, No. 1, $S_3$–$S_7$ (1950)» wird die Herstellung von 3,3′-Dihydroxy-2,2′,4,4′,6,6′-hexamethyldiphenylmethan und 3-Hydroxy-2,4,6-trimethylbenzyl-ethylether beschrieben.

Aus einem Artikel von R. Wegler und E. Regel in «Die Makromolekulare Chemie 9, 1–24 (1952)» ist die Herstellung der Verbindung 3,3′-Dihydroxy-2,2′,4,4′,6,6′-hexamethyl-dibenzylether bekannt.

Gegenstand der Erfindung sind Verbindungen der Formel I

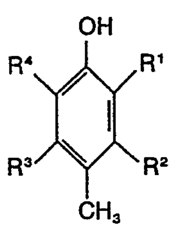

(I)

in der $R^1$ $C_1$–$C_4$-Alkyl, $R^4$ $C_1$–$C_{12}$-Alkyl, $C_5$–$C_8$-Cycloalkyl, $C_6$–$C_{10}$-Aryl, $C_7$–$C_{10}$-Aralkyl oder $C_7$–$C_{10}$-Alkaryl ist und $R^3$ Wasserstoff oder eine Gruppe der Formel V bedeutet, und $R^2$ einen der Reste der Formeln II, III, IV oder V darstellt,

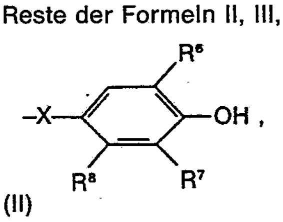

(II)

,

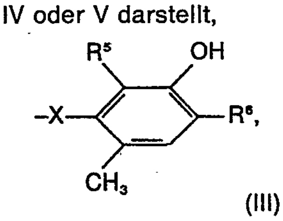

(III)

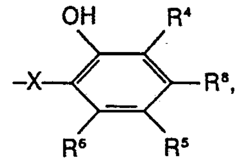

(IV)

$-CH_2OY$ (V)

wobei für $R^5$ die für $R^1$ angegebene Definition gilt und $R^5$ gleich oder verschieden von $R^1$ sein kann und für $R^6$ die für $R^4$ angegebene Definition gilt und $R^6$ gleich oder verschieden von $R^4$ sein kann, und X $-CH_2-$, $-CH_2OCH_2-$ oder $-CH_2SCH_2-$ bedeutet, und $R^7$ $C_4$–$C_{12}$-Alkyl, $C_5$–$C_8$-Cycloalkyl, $C_6$–$C_{10}$-Aryl, $C_7$–$C_{10}$-Aralkyl oder $C_7$–$C_{10}$-Alkaryl und $R^8$ Methyl oder Wasserstoff ist, und Y $C_1$–$C_{20}$-Alkyl, $C_6$–$C_{10}$-Aryl, $C_7$–$C_{10}$-Aralkyl oder $C_7$–$C_{10}$-Alkaryl ist, sowie Verbindungen der Formel VI,

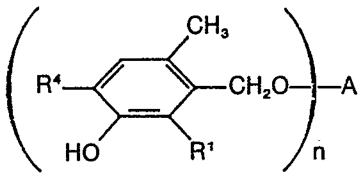

(VI)

in der $R^1$ und $R^4$ wie in Formel I definiert sind, n eine Zahl von 2–4 bedeutet und

A bei n = 2 $-C_mH_{2m}-$, worin m eine Zahl von 2 bis 12 darstellt, eine ein- oder zweimal durch –O–, –S– oder –NH– unterbrochene Gruppe $-C_mH_{2m}-$, $C_4$–$C_8$-Alkenylen, $C_4$–$C_8$-Alkinylen, Phenylen oder eine Gruppe

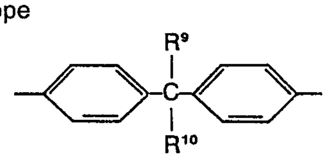

,

worin $R^9$ und $R^{10}$ unabhängig voneinander H oder $C_1$–$C_4$-Alkyl darstellen, und A ferner bei n = 3 eine der Gruppen

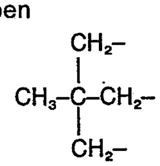

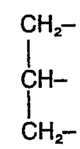

$N(CH_2CH_2-)_3-$ oder $CH_3-CH_2-C(CH_2-)_3-$ und
A bei n = 4 eine Gruppe $C(CH_2-)_4-$
bedeutet, unter Ausschluss der Verbindungen 3,3′-Dihydroxy-2,2′,4,4′-6,6′-hexamethyldiphenylmethan, 3,3′-Dihydroxy-2,2′,4,4′,6,6′-hexamethyldibenzylether und 3-Hydroxy-2,4,6-trimethylbenzyl-ethylether.

Bedeuten $R^1$, $R^4$, $R^5$, $R^6$ und $R^7$ $C_1$–$C_4$, $C_1$–$C_{12}$ bzw. $C_4$–$C_{12}$-Alkyl, so kann es sich z.B. um Methyl, Äthyl, n-Propyl, Isopropyl, n-Butyl, sec.-Butyl, tert.-Butyl, n-Pentyl, n-Hexyl, n-Octyl, 1,1,3,3-Tetramethylbutyl, n-Nonyl, Decyl oder Dodecyl handeln. $R^1$, $R^4$, $R^5$ und $R^6$ sind bevorzugt Methyl oder tert.-Butyl. Die bevorzugte Bedeutung von $R^7$ ist Methyl. Y als $C_1$–$C_{20}$-Alkyl kann zusätzlich z.B. Tetradecyl, Hexadecyl, Heptadecyl, Octadecyl oder Eicosyl darstellen. Bevorzugt ist Y eine $C_6$–$C_{18}$-Alkylgruppe.

Bedeuten $R^4$, $R^6$, $R^7$ und Y $C_6$–$C_{10}$-Aryl, so kann es sich beispielsweise um Naphthyl und bevorzugt um Phenyl handeln.

Bedeuten $R^4$, $R^6$, $R^7$ und Y $C_7$–$C_{10}$-Aralkyl, so handelt es sich z.B. um Benzyl, 1-Phenyläthyl, α,α-Dimethylbenzyl oder um 2-Phenyläthyl.

$R^4$, $R^6$ und $R^7$ sind als $C_5$–$C_8$-Cycloalkyl z.B. Cyclopentyl, Cycloheptyl, Cyclooctyl und bevorzugt Cyclohexyl.

Als $C_7$–$C_{10}$-Alkaryl sind $R^4$, $R^6$, $R^7$ und Y beispielsweise Tolyl 2,4-Dimethylphenyl, 2,6-Dimethylphenyl, 2,4-Diäthylphenyl, 2,6-Diäthylphenyl oder 4-tert.-Butylphenyl. Bevorzugt ist 2,4-Dimethylphenyl.

Bedeutet A eine Gruppe $-C_mH_{2m}-$, worin m eine Zahl zwischen 2 und 12 ist, so bedeutet m bevorzugt eine Zahl zwischen 2 und 8. Beispiele sind Äthylen, Trimethylen, Tetramethylen, Hexamethylen, Octamethylen, Nonamethylen, 2,2,4-Trimethylhexamethylen, Decamethylen, Dodecamethylen.

Ist A $C_4$–$C_8$-Alkenylen, so handelt es sich insbesondere um 2-Butenylen-1,4.

Ist A $C_4$–$C_8$-Alkinylen, so handelt es sich insbesondere um 2-Butinylen-1,4. Bevorzugt werden Verbindungen der Formel I, worin $R^2$ ein Rest der Formel II, III oder V ist, und insbesondere solche worin $R^2$ ein Rest der Formeln II oder III ist.

Bedeutet $R^2$ in Formel I ein Rest der Formel III, so bedeuten in diesem die Reste $R^5$ und $R^6$ vorzugsweise $C_1$–$C_4$ Alkyl; die Reste $R^1$ und $R^4$ sind dann bevorzugt Methyl und $R^3$ Wasserstoff.

Die bevorzugte Bedeutung von X ist $-CH_2-$.

Eine Vorzugsform der Erfindung stellen solche Verbindungen der Formel I dar, wobei in Formel I $R^1$ und $R^5$ $CH_3$, $R^4$ und $R^6$ $C_1$–$C_4$-Alkyl und $R^7$ t-Butyl, $R^8$ Wasserstoff und Y $C_6$–$C_{18}$-Alkyl bedeuten.

Eine weitere Vorzugsform der Erfindung sind Verbindungen der Formel VI, in der $R^1$ $CH_3$, $R^4$ $C_1$–$C_4$-Alkyl, n die Zahl 2 und A $-C_mH_{2m}-$, worin m eine Zahl von 2 bis 8 darstellt oder die Gruppe

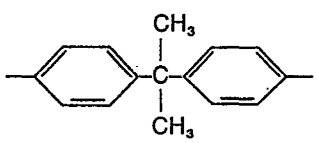

bedeuten.

Beispiele der erfindungsgemässen Verbindungen sind folgende Substanzen.

Gemäss Formel I:

3,4'-Dihydroxy-4,3',5'-tri-t-butyl-2,6-dimethyl-diphenylmethan,

3,4'-Dihydroxy-4,3'-di-t-butyl-2,6,5'-trimethyl-diphenylmethan,

3,3'-Dihydroxy-4,4'-di-t-butyl-2,2'-6,6'-tetramethyldiphenylmethan,

3,3'-Dihydroxy-4,4'-di-t-butyl-2,2',6,6'-tetramethyldibenzylsulfid,

3,3'-Dihydroxy-4,4'-di-t-butyl-2,2',6,6'-tetramethyldibenzyläther,

3-Hydroxy-4-t.-butyl-2,6-dimethylbenzyl-n-octadecyläther,

3-Hydroxy-4-t-butyl-2,6-dimethylbenzyl-isopropyläther,

3,4'-Dihydroxy-3',5'-di-t-butyl-2,4,6-trimethyl-diphenylmethan,

Gemäss Formel VI:

1,2-Di(3-hydroxy-4-t-butyl-2,6-dimethylbenzyl-oxy)-äthan,

1,6-Di-(3-hydroxy-4-i-amyl-2,6-dimethylbenzyl-oxy)-n-hexan, Pentaerythrit-tetra-(3-hydroxy-4-t-butyl-2,6-dimethylbenzyl-äther),

Neopentylglykol-di-(3-hydroxy-4-cyclohexyl-2,6-dimethylbenzyl-äther).

Die erfindungsgemässen mehrkernigen Stabilisatoren der Formel I sind nach an sich bekannten Benzylierungsreaktionen an gehinderten Phenolen erhältlich, wobei ein Phenol der Formel VII

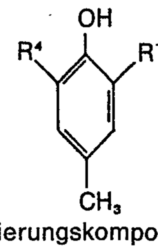

mit einer Benzylierungskomponente der Formeln VIII, IX oder X umgesetzt wird

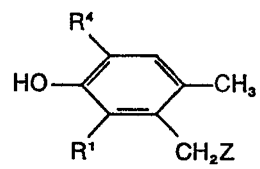

(VIII)

(Z bedeutet Cl, Br, J, OH; vorzugsweise Cl)

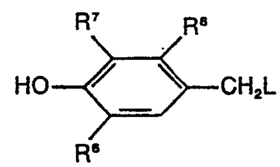

(IX)

(L bedeutet OH, $-OC_bH_{2b+1}$, $-N(C_bH_{2b+1})_2$; b = 1–4.)

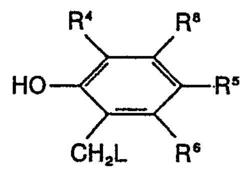

(X)

wobei das molare Verhältnis der Verbindung der Formel VII zu den Verbindungen der Formeln VIII, IX oder X ungefähr 1:1 ist.

Die Reaktionen werden gegebenenfalls in Gegenwart von Basen (z.B. t-Amine, Alkalien, Erdalkalien), gegebenenfalls aber auch in Gegenwart von sauren Katalysatoren, wie $H_2SO_4$, HCl, p-Toluolsulfonsäure, $BF_3$-Ätherat, $ZnCl_2$, $AlCl_3$, und gegebenenfalls in Gegenwart eines inerten Lösungsmittels (z.B. Alkane, Aromaten, Äther, DMF, DMA) durchgeführt. Die Verbindungen der Formeln VIII, IX und X können bei der Reaktion auch im Überschuss eingesetzt werden.

Die Äther der speziellen Formel I

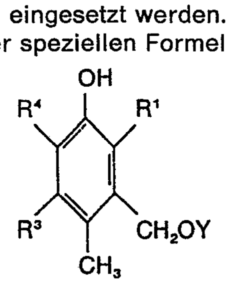

sowie die Äther gemäss Formel VI sind nach bekannten Verfahren erhältlich, beispielsweise durch Benzylieren von Alkoholen Y–OH mit den Benzylierungskomponenten der Formel VIII. Ist $R^3$ eine Gruppe der Formel V, so wird die Reaktion mit etwa zwei Mol des Alkohols Y–OH pro Mol der Verbindung der Formel VII durchgeführt, während sonst äquimolare Mengen zur Umsetzung gebracht werden.

Als Ausgangsstoffe für die Herstellung der erfindungsgemässen Verbindungen werden bekannte Substanzen verwendet, deren Herstellung dem Fachmann ebenfalls bekannt ist.

Ein weiterer Gegenstand der Erfindung ist die Verwendung der im Anspruch 11 definierten Verbindungen der Formeln IA und VI als Stabilisatoren für organisches Material gegen dessen Schädigung durch Einwirkung von Sauerstoff, Wärme, Licht und energiereiche Strahlung, wie z. B. β- und γ-Strahlung.

Vorzugsformen dieser erfindungsgemässen Verwendung sind die Verwendung der Verbindungen als Stabilisatoren in organischen Polymeren, insbesondere in Pfropfpolymeren auf der Basis Acrylnitril, Butadien, Styrol (ABS) oder in schlagfestem Polystyrol.

Weitere Beispiele für organisches Material, welches mit den Verbindungen der Formeln IA und VI vorteilhaft stabilisiert werden kann, sind:

1. Polymere von Mono- und Diolefinen, beispielsweise Polyäthylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z. B. von Cyclopenten oder Norbornen.

2. Mischungen der unter 1) genannten Polymeren, z.B. Mischungen von Polypropylen mit Polyäthylen oder mit Polyisobutylen.

3. Copolymere von Mono- und Diolefinen untereinander oder mit anderen Vinylmonomeren, wie z.B.
Äthylen-Propylen-Copolymere,
Propylen-Buten-1-Copolymere,
Propylen-Isobutylen-Copolymere,
Äthylen-Buten-1-Copolymere,
Propylen-Butadien-Copolymere,
Isobutylen-Isopren-Copolymere,
Äthylen-Alkylacrylat-Copolymere,
Äthylen-Alkylmethacrylat-Copolymere,
Äthylen-Vinylacetat-Copolymere oder
Äthylen-Acrylsäure-Copolymere und deren Salze (Ionomere), sowie Terpolymere von Äthylen mit Propylen und einem Dien, wie Hexadien, Dicyclopentadien oder Äthylidennorbornen.

4. Polystyrol.

5. Copolymere von Styrol oder α-Methylstyrol mit Dienen oder Acrylderivaten, wie z.B. Styrol-Butadien, Styrol-Acrylnitril-Methylacrylat; Mischungen von hoher Schlagzähigkeit aus Styrol-Copolymeren und einem anderen Polymer wie z.B. einem Polyacrylat, einem Dien-Polymeren oder einem Äthylen-Propylen-Dien-Terpolymeren; sowie Block-Copolymere des Styrols, wie z.B. Styrol-Butadien-Styrol, Styrol-Isopren-Styrol, Styrol-Äthylen/Butylen-Styrol oder Styrol-Äthylen/Propylen-Styrol.

6. Pfropfcoplymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw. Alkylmethacrylate und Polybutadien, Styrol und Acrylnitril auf Äthylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie z.B. als sogenannte ABS, MBS, oder AES-Polymere bekannt sind.

7. Halogenhaltige Polymere, wie z.B. Polychloropren, Chlorkautschuk, chloriertes oder chlorsulfoniertes Polyäthylen, Epichlorhydrinhomo- und copolymere, insbesondere Polymere aus halogenhaltigen vinylverbindungen, wie z.B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere, oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Äthern, wie Polyalkylenglykole, Polyäthylenoxyd, Polypropylenoxyd oder deren Copolymere mit Bisglycidyläthern.

12. Polyacetale, wie Polyoxymethylen, sowie solche Polyoxymethylene, die Comonomere wie z.B. Äthylenoxyd enthalten.

13. Polyphenylenoxide und -sulfide, sowie Mischungen von Polyphenylenoxiden mit Polystyrol.

14. Polyurethane, die sich von Polyäthern, Polyestern und Polybutadienen mit endständigen Hydroxylgruppen einerseits und aliphatischen oder aromatischen Polyisocyanaten andererseits ableiten sowie deren Vorprodukte (Polyisocyantate, Polyole, Präpolymere).

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid, sowie deren Copolymere mit Polyäthern wie z.B. mit Polyäthylenglykol Polypropylenglykol oder Polytetramethylenglykol.

16. Polyharnstoffe, Polyimide und Polyamidimide.

17. Polyester, die sich von Dicarbonsäuren und Diolen und/oder von Hydroxycarbonsäuren oder den entsprechenden Lactonen ableiten, wie Polyäthylenterephthalat, Polybutylenterephthalat, Poly-1,4-dimethylolcyclohexanterephthalat, Poly-[2,2-bis(4-hydroxyphenyl)-propanterephthalat, Polyhydroxybenzoate, sowie Block-Polyäther-ester, die sich von Polyäthylen mit Hydroxyengruppen, Dialkoholen und Dicarbonsäuren ableiten.

18. Polycarbonate,

19. Polysulfone und Polyäthersulfone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die ich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycicyläthern oder cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseäther, wie Methylcellulose.

27. Natürliche und synthetische organische Stoffe, die reine monomere Verbindungen oder Mischungen von solchen darstellen, beispielsweise Mineralöle, tierische und pflanzliche Fette, Öle und Wachse, oder Öle, Wachse und Fette auf Basis synthetischer Ester (z.B. Phthalate, Adipate, Phosphate oder Trimellithate), sowie Abmischungen synthetischer Ester mit Mineralölen in beliebigen Gewichtsverhältnissen, wie sie z.B. als Weichmacher für Kunststoffe oder als Spinpräparationen Anwendung finden, sowie deren wässrigen Emulsionen.

28. Wässrige Emulsionen natürlicher oder synthetischer Kautschuke wie z.B. Naturkautschuk-Latex oder Latices von carboxylierten Styrol-Butadien-Copolymeren.

Von besonderer Bedeutung ist die Stabilisierung von Styrolpolymeren und Elastomeren. Die erfindungsgemäss stabilisierten Polymeren weisen ein ausgezeichnetes Farbverhalten auf und es besteht beste Verträglichkeit dieser Stabilisatoren mit den Polymeren.

Die Verbindungen der Formeln IA und VI sind auch besonders vorteilhaft als Stabilisatoren für Schmiermittel geeignet.

Die Stabilisatoren werden den Kunststoffen in einer Konzentration von 0,01 bis 5 Gew.-%, berechnet auf das zu stabilisierende Material, zugesetzt. Vorzugsweise werden 0,1 bis 2,0, besonders bevorzugt 0,2 bis 0,6 Gew.-% der Verbindungen, berechnet auf das zu stabilisierende Material, in dieses eingearbeitet.

Die Einarbeitung kann nach der Polymerisation erfolgen, beispielsweise durch Einmischen der Verbindungen und gegebenenfalls weiterer Additive in die Schmelze nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels.

Die durch Zusatz von 0.01 bis 5 Gew.-% einer Verbindung der Formel IA oder VI stabilisierten Kunststoffe können gegebenenfalls noch andere bekannte und übliche Zusätze enthalten. Die so stabilisierten Kunststoffe können in verschiedenster Form angewendet werden z.B. als Folien, Fasern, Bändchen, Profile oder als Bindemittel für Lacke, Klebemittel oder Kitte.

Als weitere Additive, mit denen zusammen die erfindungsgemäss verwendbaren Stabilisatoren eingesetzt werden können, sind beispielsweise zu nennen:

1. Antioxidantien
1.1 Alkylierte Monophenole
2,6-Di-tert.butyl-4-methylphenol
2-Tert.butyl-4,6-dimethylphenol
2,6-Di-tert.butyl-4-äthylphenol
2,6-Di-tert.-butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol
2,6-Diphenyl-4-octadecyloxyphenol

1.2. Alkylierte Hydrochinone
2,6-Di-tert.butyl-4-methoxyphenol
2,5-Di-tert.butyl-hydrochinon
2,5-Di-tert.amyl-hydrochinon

1.3. Hydroxylierte Thiodiphenyläther
2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)

1.4. Alkyliden-Bisphenole
2,2'-Methylen-bis-(6-tert.butyl-4-methylphenol)
2,2'-Methylen-bis-(6-tert.butyl-4-äthylphenol)
2,2'-Methylen-bis-[4-methyl-6-(α-methylcyclohexyl)-phenol]
2,2'-Methylen-bis-(4-methyl-6-cyclohexylphenol)

2,2'-Methylen-bis-(6-nonyl-4-methylphenol)
2,2'-Methylen-bis-(4,6-di-tert.butylphenol)
2,2'-Äthyliden-bis(4,6-di-tert.butylphenol)
2,2'-Äthyliden-bis-(6-tert.butyl-4-isobutylphenol)
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methyl-phenyl)-butan
2,6-Di-(3-tert.butyl-5-methyl-2-hydroxybenzyl)-4-methylphenol
1,1,3-Tris-(5-tert.butyl-4-hydroxy-2-methyl-phenyl)-butan
1,1-Bis-(5-tert.butyl-4-hydroxy-2-methyl-phenyl)-3-n-dodecylmercaptobutan
Äthylenglycol-bis-[3,3-bis-(3'-tert.butyl-4-hydroxyphenyl)-butyrat]
Di-(3-tert.butyl-4-hydroxy-5-methylphenyl)-dicyclopentadien
Di-[2-(3'-tert.butyl-2'-hydroxy-5'-methylbenzyl)-6-tert.butyl-4-methyl-phenyl]-terephthalat.

1.5. Benzylverbindungen
1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoäthylester,
Calcium-salz.

1.6. Acylaminophenole
4-Hydroxy-laurinsäureanilid
4-Hydroxy-stearinsäureanilid
2,4-Bis-octylmercapto-6-(3,5-di-tert.butyl-4-hydroxyanilino)-S-triazin

1.7. Ester der β-(3,5-Di-tert.butyl-4-hydroxyphenyl)-propionsäure
mit ein- oder mehrwertigen Alkoholen, wie z.B. mit
Methanol
Octadecanol
1,6-Hexandiol
Neopentylglycol
Thiodiäthylenglycol
Diäthylenglycol
Triäthylenglycol
Pentaerythrit
Tris-hydroxyäthyl-isocyanurat
Di-hydroxyäthyl-oxalsäurediamid

1.8. Ester der β(5-tert.butyl-4-hydroxy-3-methylphenyl)-propionsäure
mit ein- oder mehrwertigen Alkoholen, wie z.B. mit
Methanol
Octadecanol
1,6-Hexandiol
Neopentylglycol
Thiodiäthylenglycol
Diäthylenglycol
Triäthylenglycol
Pentaerythrit
Tris-hydroxyäthyl-isocyanurat
Di-hydroxyäthyl-oxalsäurediamid

1.9. Amide der β-(3,5-Di-tert.butyl-4-hydroxyphenyl)-propionsäure,
wie z.B.
N,N'-Di-(3,5-di-tert.butyl-4-hydroxyphenyl-propionyl)-hexamethylendiamin
N,N'-Di-(3,5-di-tert.butyl-4-hydroxyphenyl-propionyl)-trimethylendiamin
N,N'-Di-(3,5-di-tert.butyl-4-hydroxyphenyl-propionyl)-hydrazin

2. UV-Absorber und Lichtschutzmittel

2.1. 2-(2'-Hydroxyphenyl)-benztriazole, wie z.B. das
5'-Methyl-, 3',5'-Di-tert.butyl-,
5'-Tert.butyl-, 5'-(1,1,3,3-Tetramethylbutyl)-,
5-Chlor-3',5'-di-tert.butyl-,
5-Chlor-3'-tert.butyl-5'-methyl-,
3'-sec-Butyl-5'-tert.butyl-, 4'-Octoxy-,
3',5'-Di-tert.amyl-,

2.2. 2-Hydroxybenzophenone, wie z.B. das
4-Hydroxy-, 4-Methoxy-, 4-Octoxy-, 4-Decyloxy-,
4-Dodecyloxy-, 4-Benzyloxy-, 4,2',4'-Trihydroxy-,
2'-Hydroxy-4,4'-dimethoxy-Derivat.

2.3. Ester von gegebenenfalls substituierten Benzoesäuren, wie z.B.
4-Tert.butyl-phenylsalicylat, Phenylsalicylat,
Octylphenylsalicylat, Dibenzoylresorcin,
Bis-(4-tert.butylbenzoyl)-resorcin,
Benzoylresorcin,
3,5-Di-tert.butyl-4-hydroxybenzoesäure-2,4-di-tert.butyl-phenhylester.

2.4. Acrylate, wie z.B.
α-Cyan-β,β-diphenylacrylsäure-äthylester bzw. -isooctylester-
α-Carbomethoxy-zimtsäuremethylester,
α-Cyano-β-methyl-p-methoxy-zimtsäuremethylester bzw. -butylester.
α-Carbomethoxy-p-methoxy-zimtsäure-emthylester.
N-(β-Carbomethoxy-β-cyanovinyl)-2-methyl-indolin.

2.5. Nickelverbindungen, wie z.B.
Nickelkomplexe des 2,2'-Thio-bis-[4-(1,1,3,3-tetramethylbutyl)-phenols],
wie der 1:1- oder der 1:2-Komplex, gegebenenfalls mit zusätzlichen Liganden wie n-Butylamin, Triäthanolamin oder N-Cyclohexyl-diäthanolamin,
Nickeldibutyldithiocarbamat, Nickelsalze von

4-Hydroxy-3,5-di-tert.butylbenzyl-phosphonsäure-monoalkylestern wie vom Methyl- oder Äthylester, Nickelkomplexe von Ketoximen wie von 2-Hydroxy-4-methyl-phenyl-undecyl-ketonoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B.
Bis-(2,2,6,6-tetramethyl-piperidyl)-sebacat
Bis-(1,2,2,6,6-pentamethylpiperidyl)-sebacat
n-Butyl-3,5-di-tert.butyl-4-hydroxybenzyl-malonsäure-bis(1,2,2,6,6-pentamethyl-piperidyl)-ester
Kondensationprodukt aus 1-Hydroxyäthyl-2,2,6,6-Tetramethyl-4-hydroxypiperidin und Bernsteinsäure Kondensationsprodukt aus N,N'-(2,2,6,6-Tetramethylpiperidyl)-hexamethylendiamin und 4-tert.octylamino-2,6-dichlor-1,3,5-s-triazin.
Tris-(2,2,6,6-tetramethylpiperidyl)-nitrilo-triacetat.

2.7. Oxalsäurediamide, wie z.B.
4,4'-Di-octyloxy-oxanilid,
2,2'-Di-octyloxy-5,5'-di-tert.butyl-oxanilid,
2,2'-Di-dodecyloxy-5,5'-di-tert.butyl.oxanilid,
2-Äthoxy-2'-äthyl-oxanilid,
N,N'-Bis-(3-di-methylaminopropyl)-oxalamid,
2-Äthoxy-5-tert.butyl-2'-äthyl-oxanilid und dessen Gemisch mit 2-Äthoxy-2'-äthyl-5,4'-di-tert.butyl-oxanilid,
Gemische von ortho- und para-Methoxy- sowie von o- und p-Äthoxy-di-substituierte Oxaniliden.

3. Metalldesaktivatoren, wie z.B.
N,N'-Diphenyloxalsäurediamid,
N-Salicylal-N'-salicyloylhydrazin,
N,N'-Bis-salicyloylhydrazin,
N,N'-Bis-(3,5-di-tert.butyl-4-hydroxyphenyl-propionyl)-hydrazin,
3-Salicyloylamino-1,2,4-triazol,
Bis-benzyliden-oxalsäuredihydrazid.

4. Phosphite und Phosphonite, wie z.B.
Triphenylphosphit, Diphenylalkylphosphite,
Phenyldialkylphosphite,
Tri-(nonylphenyl)-phosphit,
Trilaurylphosphit, Trioctadecylphosphit,
Distearyl-pentaerythrit-diphosphit,
Tris-(2,4-di-tert.butylphenyl)-phosphit,
Diisodecyl-pentaerythrit-diphosphit,
Di-(2,4-di-tert.butylphenyl)-pentaerythrit-diphosphit,
Tristearyl-sorbit-triphosphit,
Tetrakis-(2,4-di-tert. butylphenyl)-4,4'-biphenylen-diphosphonit.

5. Peroxidzerstörende Verbindungen, wie z.B. Ester der β-Thio-di-propionsäure, beispielsweise der Lauryl-, Stearyl-, Myristyl- oder Tridecylester, Mercaptobenzimidazol, das Zinksalz des 2-Mercaptobenzimidazols,
Zink-dibutyl-dithiocarbamat,
Dioctadecyldisulfid,
Pentaerythrit-tetrakis (β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert-Butylbenzoesäure, Adipinsäure, Diphenylessigsäure.

9. Füllstoffe und Verstärkungsmittel, wie z.B. Calciumcarbonate, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Beispiele

A) Herstellung

Beispiel 1:

3,4'-Dihydroxy-4,3',5'-tri-t-butyl-2,6-dimethyl-diphenylmethan.

21,5 g 2,4-Dimethyl-6-t.butyl-phenol werden in 60 ml Methanol gelöst und mit 11 g 80%iger Schwefelsäure versetzt. Dann werden bei ca. 70 °C (Rückfluss) unter Rühren und Stickstoff innerhalb 6 Stunden 30 g 4-Methoxymethyl-2,6-di-t-butyl-phenol zugegeben. Das Reaktionsgemisch wird weitere 6 Stunden bei 70 °C gehalten. Nach dem Abkühlen kristallisiert das Reaktionsprodukt in farblosen Kristallen aus. Es wird abgenutscht und durch Digerieren mit Hexan, Abnutschen, Digerieren mit NaOH-haltigem Wasser und abermaligem Abnutschen gereinigt. Smp. 144 °C (aus Methanol).

Beispiel 2:

3,4'-Dihydroxy-4,3'-di-t-butyl-2,6,5'-trimethyl-diphenylmethan.

Ersetzt man das 4-Methoxymethyl-2,6-di-t-butyl-phenol von Beispiel 1 durch die aliquote Menge des 4-Methoxymethyl-2-methyl-6-t-butyl-phenols, so erhält man bei analoger Arbeitsweise das 3,4'-Dihydroxy-4,3'-di-t-butyl-2,6,5'-trimethyl-diphenylmethan vom Smp. 122 °C.

Beispiel 3:

Ersetzt man das 2,4-Dimethyl-6-t-butylphenol von Beispiel 1 durch die aliquote Menge des 2,4,6-Trimethylphenols, so erhält man bei analoger Arbeitsweise das 3,4'-Di-hydroxy-3',5'-di-t-butyl-2,4,6-trimethyl-diphenylmethan vom Smp.: 145 °C.

Beispiel 4:

3,3'-Di-hydroxy-4,4'-di-t-butyl-2,2',6,6'-tetramethyl-diphenylmethan.

22,6 g 6-t-Butyl-2,4-dimethyl-3-chlormethyl-phenol[1]

und 17,8 g 2-t-Butyl-4,6-dimethyl-phenol wurden nach Zugabe von 0,5 g wasserfreiem Zinkchlorid 10 Std. auf 60 °C erwärmt (Rühren, Stickstoff). Anschliessend wird das Produkt aus der Toluollösung nach Wasserwäsche, Abdampfen des Lösungsmittels und Anreiben mit Hexan kristallin erhalten. Smp. 150 °C.

Beispiel 5:

3,3'-Dihydroxy-4,4'-di-t-butyl-2,2',6,6'-tetramethyl-dibenzylsulfid.

22,6 g 6-t-Butyl-2,4-dimethyl-chlormethyl-phenol wurden unter Zugabe von 10,1 g Triäthylamin in 50 ml Toluol gelöst und bei 20–30 °C ca. 2,5 g Schwefelwasserstoff unter Rühren langsam eingeleitet. Nach 20 Std. Rühren bei 20–30 °C wird die Toluollösung mit Wasser neutral gewaschen und das Produkt durch Säulenchromatographie an Kieselgel (Toluol) vom ebenfalls gebildeten entsprechenden Benzylmercaptan, Smp. – 60 °C abgetrennt.

Weisse Kristalle vom Smp. 130 °C.

Beispiel 6:

3,3'-Dihydroxy-4,4'-di-t-butyl-2,2',6,6'-tetramethyl-dibenzyläther.

22,6 g 6-t-Butyl-2,4-dimethyl-3-chlormethyl-phenol

werden in 100 ml siedendem Wasser unter Zugabe von 12,6 g wasserfreiem Natriumcarbonat 4 Stunden am Rückfluss unter Rühren erhitzt. Nach Abkühlen und Zugabe von Methylenchlorid wird die wässrige Phase abgetrennt, die organische Phase mit Wassser gewaschen, getrocknet und eingedampft. Aus dem Rückstand erhält man durch Säulenchromatographie an Kieselgel (Toluol:Aceton = 96:4) das Produkt dieses Beispiels. Weisse Kristalle vom Smp. 134 °C.

(Als Nebenprodukt entsteht der entsprechende Benzylalkohol, Smp. 148 °C).

Beispiel 7:

3-Hydroxy-4-t-butyl-2,6-dimethylbenzyl-n-octadecyl-äther.

11.3 g 6-t-Butyl-2,4-dimethyl-3-chlormethyl-phenol,

13,5 g n-Octadecanol, 70 ml Dimethylacetamid und 5,5 g Triäthylamin werden unter Zusatz von 0,2 g Kaliumjodid 25 Stunden auf 100 °C erhitzt (Rühren). Der Ansatz wird dann mit Methylenchlorid und Wasser versetzt, die organische Phase abgetrennt, mit Wasser gewaschen, getrocknet und eingedampft. Das Produkt kristallisiert aus Acetonitril. Smp.: 52 °C

[1] Herstellung siehe «Makromolek. Chemie» Bd. 9, S. 21–22 (1952)

Beispiel 8:

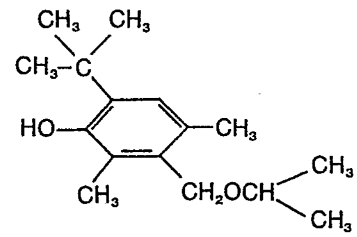

Verwendet man als Alkohol-Komponente Isopropanol und verfährt analog Beispiel 7, so erhält man den entsprechenden Isopropyläther mit Smp. 50 °C.

Beispiel 9:

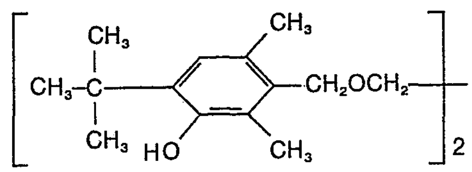

Verwendet man als Alkohol-Komponente Äthylenglykol und arbeitet analog Beispiel 7, so erhält man den entsprechenden Äthylenglykoldiäther mit Smp. 143 °C, welcher eine Verbindung der Formel VI darstellt.

Beispiel 10:

100 Gewichtsteile unstabilisiertes ABS-Pulver werden mit verschiedenen in den Tabellen I und II angegebenen Stabilisatoren gemischt.

Die erhaltenen Gemische werden auf einem Zweiwalzenstuhl während 5 Minuten bei maximal 170 °C compoundiert und die Felle anschliessend abgezogen. Die Rohfelle werden auf einer hydraulischen Laborpresse bei 180 °C während 6 Minuten zu 1 mm dicken Platten verpresst, aus welchen Prüflinge der Dimension 50 × 20 mm gestanzt werden.

Die Prüfung der Wirksamkeit der den Prüflingen zugesetzten Stabilisatoren wird durch Hitzealterung in einem Umluftofen bei 180 °C vorgenommen. Als Kriterium für die während der Alterung eingetretene Schädigung (Oxidation) dient das Infrarot-Absorptionsspektrum der Oberfläche, welches durch Reflexions-Spektroskopie (ATR) erhalten wird. Insbesondere wird die Zunahme der Carbonylextinktion ($1720\ cm^{-1}$) als Funktion der Zeit verfolgt und mit einer konstant bleibende Absorptionsbande ($1455\ cm^{-1}$) verglichen. Dabei gilt als Mass für den Abbau:

$$V = \frac{\text{Opt. Dichte bei } 1720\ cm^{-1}\ (>C = O)}{\text{Opt. Dichte bei } 1455\ cm^{-1}\ (>CH_2)}$$

Als willkürlicher Endpunkt wird die Zeit gewählt, nach welcher V den Wert 0,1 erreicht ($t_{0,1}$).

Tabelle I
Prüfung in ABS ohne Synergist

| Stabilisator nach Herst. Beisp. Nr. 0,25 Gew.-% | Ofenalterung 180 °C | | | | | | |
|---|---|---|---|---|---|---|---|
| | | Y.I. ASTM D 1925 nach Min. Ofenalterung | | | | | |
| | $^{t}$ 0,1 | 0 | 30 | 60 | 90 | 120 | 150 |
| 1 | 25' | 15 | 42 | 66 | 77 | 86 | |
| 3 | 75' | 18,5 | 31,0 | 37,6 | 65,9 | 78,6 | 87,8 |
| 5 | 49' | 13,9 | 25,0 | 33,2 | 55,0 | 66,9 | 80,3 |
| 6 | 90' | 16,4 | 26,4 | 30,1 | 46,4 | 64,4 | 78,0 |
| 7 | 43' | 14,0 | 24,1 | 46,2 | 63,2 | 73,8 | 82,8 |
| ohne Stabilisator | 7' | 17 | 53 | 73 | 83 | | |

Tabelle II
Prüfung in ABS mit dem Synergist DLTDP (Dilaurylthiodipropionat)

| Stabilisator nach Herst. Beisp. Nr. + DLT DP 0,25 Gew.% Stab. 0,5 Gew.% DLTDP | Ofenalterung 180 °C | | | | | | |
|---|---|---|---|---|---|---|---|
| | | Y.I. ASTM nach Min. Ofenalterung | | | | | |
| | $^{t}$ 0,1 | 0 | 30 | 60 | 90 | 120 | 150 |
| 1 | 55' | 15 | 26 | 40 | 73 | 90 | |
| 3 | 86' | 18,8 | 29,6 | 35,6 | 51,4 | 74,5 | 90,9 |
| 5 | 105' | 14,1 | 24,0 | 27,6 | 32,5 | 45,4 | 52,0 |
| 6 | 154' | 15,6 | 25,3 | 30,2 | 33,9 | 36,8 | 45,0 |
| 7 | 60' | 13,1 | 22,5 | 29,5 | 37,6 | 49,1 | 71,1 |
| ohne Stabilisator | 7' | 17 | 53 | 73 | 83 | | |

Beispiel 11:

Ein schlagfestes Polystyrol mit 8 Gew.-% Polybutadien-Gehalt (high-cis) und mit 0,035 Gew.-% 2,6-Di-tert.-butyl-p-cresol als Grundstabilisator, 0,05 Gew.-% Zinkstearat als Gleitmittel sowie 0,1 Gew.-% eines der erfindungsgemässen Antioxidantien (in den nachfolgenden Tabellen III und IV jeweils mit der Nummer des entsprechenden Herstellungsbeispiels gekennzeichnet), wird zweimal bei 220 °C extrudiert und das erhaltene Granulat bei 185 °C in 3 Minuten zu 2 mm dicken Prüfplättchen verpresst.

Die Prüflinge werden einer Ofenalterung im Umluftofen unterzogen und

a) der Yellowness Index gemäss ASTM D 1925 bei 80 °C (Messung der Prüflinge nach 0, 250, 500, 750 und 1000 Stunden) und bei 160 °C (Messung der Prüflinge nach 0, 60, 90, 120 und 180 Minuten) bestimmt. Die Resultate sind in Tabelle III wiedergegeben.

b) die Schlagzähigkeit (SZ) in Kp. $cm/cm^2$ nach Alterung bei 160 °C (Messung der Prüflinge nach 30, 60, 120, 150, 180, 240, 300, 360, 420, 480 Minuten) bestimmt. Letztere Resultate sind in Tabelle IV zusammengestellt.

Tabelle III

| Stab. nach Herst. | Y.I. bei 80 °C nach Stunden | | | | | Y.I. bei 160 °C nach Minuten | | | | |
|---|---|---|---|---|---|---|---|---|---|---|
| Beisp. Nr. | 0 | 250 | 500 | 750 | 1000 | 0 | 60 | 90 | 120 | 180 |
| Ohne Stab. | 0,5 | 18 | 24 | 36 | 48 | 0 | 17 | 37 | 47 | 73 |
| 1 | 0,8 | 8 | 16 | 23 | | 1 | 13 | 13 | 21 | 47 |
| 5 | 0,3 | 13 | 18 | 27 | 39 | 0 | 16 | 24 | 25 | 36 |
| 6 | 1,6 | 13 | 18 | 26 | 32 | 2 | 15 | 19 | 27 | 33 |
| 7 | 0,2 | 12 | 16 | 22 | 30 | 0 | 14 | 22 | 22 | 34 |

Tabelle IV

| Stab. nach Herst. Beisp. Nr. | SZ nach Alterung bei 160 °C nach Minuten | | | | | | | | | |
|---|---|---|---|---|---|---|---|---|---|---|
| | 30 | 60 | 120 | 150 | 180 | 240 | 300 | 360 | 420 | 480 |
| Ohne Stab. | 10,4 | | | | | | | | | |
| 1 | X | X | 16 | | | | | | | |
| 3 | X | X | X | 16 | 12 | 7 | | | | |
| 5 | X | X | X | X | X | X | X | X | 11,5 | |
| 6 | X | X | X | X | X | X | X | X | 9,7 | |
| 7 | X | X | X | X | X | X | 10,4 | | | |

X bedeutet: Prüfling nicht gebrochen

**Patentansprüche**

1. Verbindungen der Formel I

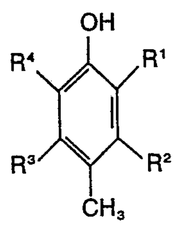

(I)

in der $R^1$ $C_1$–$C_4$-Alkyl, $R^4$ $C_1$–$C_{12}$-Alkyl, $C_5$–$C_8$-Cycloalkyl, $C_6$–$C_{10}$-Aryl, $C_7$–$C_{10}$-Aralkyl oder $C_7$–$C_{10}$-Alkaryl ist und $R^3$ Wasserstoff oder eine Gruppe der Formel V bedeutet, und $R^2$ einen der Reste der Formeln II, III, IV oder V darstellt,

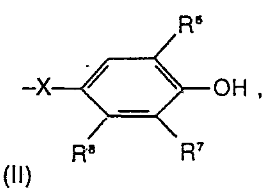

(II)

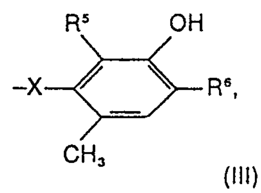

(III)

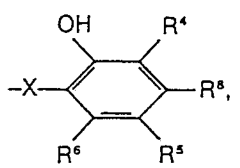

(IV)

$-CH_2OY$

(V)

wobei für $R^5$ die für $R^1$ angegebene Definition gilt und $R^5$ gleich oder verschieden und $R^1$ sein kann und für $R^6$ die für $R^4$ angegebene Definition gilt und $R^6$ gleich oder verschieden von $R^4$ sein kann, und X $-CH_2-$, $-CH_2OCH_2-$ oder $-CH_2SCH_2-$ bedeutet, und $R^7$ $C_4$–$C_{12}$-Alkyl, $C_5$–$C_8$-Cycloalkyl, $C_6$–$C_{10}$-Aryl, $C_7$–$C_{10}$-Aralkyl oder $C_7$–$C_{10}$-Alkaryl und $R^8$ Methyl oder Wasserstoff ist, und Y $C_1$–$C_{20}$-Alkyl, $C_6$–$C_{10}$-Aryl, $C_7$–$C_{10}$-Aralkyl oder $C_7$–$C_{10}$-Alkaryl ist, sowie Verbindungen der Formel VI,

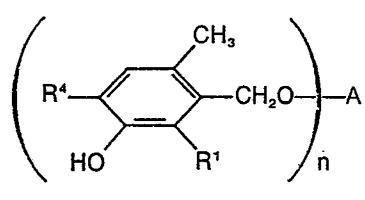

(VI)

in der $R^1$ und $R^4$ wie in Formel I definiert sind, n eine Zahl von 2–4 bedeutet und A bei n = 2 $-C_mH_{2m}-$, worin m eine Zahl von 2 bis 12 darstellt, eine ein- oder zweimal durch –O–, –S– oder –NH– unterbrochene Gruppe $-C_mH_{2m}-$, $C_4$–$C_8$-Alkenylen, $C_4$–$C_8$-Alkinylen, Phenylen oder eine Gruppe

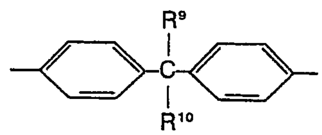

,

worin $R^9$ und $R^{10}$ unabhängig voneinander H oder $C_1$–$C_4$-Alkyl darstellen und A ferner bei n = 3 eine der Gruppen

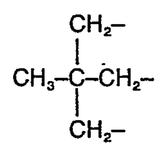

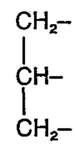

$N(CH_2CH_2-)_3-$ oder $CH_3-CH_2-C(CH_2-)_3-$ und A bei n = 4 eine Gruppe $C(CH_2-)_4-$ bedeutet, unter Ausschluss der Verbindungen 3,3′-Dihydroxy-2,2′,4,4′,-6,6′-hexamethyldiphenylmethan, 3,3′-Dihydroxy-2,2′,4,4′,6,6′-hexamethyldibenzylether und 3-Hydroxy-2,4,6-trimethylbenzyl-ethylether.

2. Verbindungen gemäss Anspruch 1 der Formel I.

3. Verbindungen gemäss Anspruch 1 der Formel VI.

4. Verbindungen gemäss Anspruch 1 oder Formel I, worin $R^3$ Wasserstoff ist.

5. Verbindungen gemäss Anspruch 1 oder Formel I, worin $R^2$ eine Gruppe der Formeln II, III oder V ist.

6. Verbindungen gemäss Anspruch 1 der Formel I, worin $R^2$ eine Gruppe der Formeln II oder III ist.

7. Verbindungen gemäss Anspruch 1 oder Formel I, wobei X $-CH_2-$ bedeutet.

8. Verbindungen gemäss Anspruch 1 der Formel I, dadurch gekennzeichnet, dass in Formel I $R^1$ und $R^5$ $CH_3$, $R^4$ und $R^6$ $C_1$–$C_4$-Alkyl sind, und $R^7$ t-Butyl ist und $R^8$ Wasserstoff bedeutet und Y $C_5$–$C_{18}$-Alkyl ist.

9. Verbindungen gemäss Anspruch 1 der Formel I, worin $R^1$ und $R^4$ Methyl und $R^3$ Wasserstoff bedeuten und $R^2$ eine Gruppe der Formel III ist, worin $R^5$ und $R^6$ $C_1$–$C_4$ Alkyl bedeuten.

10. Verbindungen gemäss Anspruch 1 der Formel VI, dadurch gekennzeichnet, dass $R^1$ $CH_3$, $R^4$ Alkyl, n die Zahl 2 und A $-C_mH_{2m}-$, worin m eine Zahl von 2–8 darstellt, oder die Gruppe

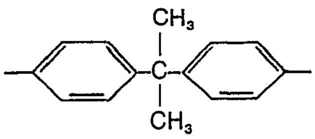

bedeuten.

11. Verwendung von Verbindungen der Formel IA,

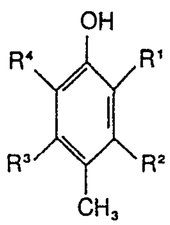

(IA)

in der $R^1$ $C_1$–$C_4$-Alkyl, $R^4$ $C_1$–$C_{12}$-Alkyl, $C_5$–$C_8$-Cycloalkyl, $C_6$–$C_{10}$-Aryl, $C_7$–$C_{10}$-Aralkyl oder $C_7$–$C_{10}$-Alkaryl ist und $R^3$ Wasserstoff oder eine Gruppe der Formel V bedeutet, und $R^2$ einen der Reste der Formeln II, III, IV oder V darstellt,

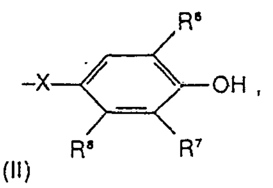

(II)

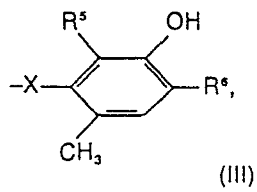

(III)

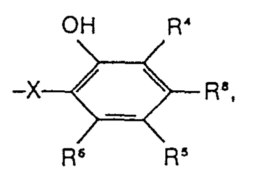

(IV)

$-CH_2OY$

(V)

wobei für $R^5$ die für $R^1$ angegebene Definition gilt und $R^5$ gleich oder verschieden von $R^1$ sein kann und für $R^6$ die für $R^4$ angegebene Definition gilt und $R^6$ gleich oder verschieden von $R^4$ sein kann, und X $-CHZ_2-$, $-CH_2OCH_2-$ oder $-CH_2SCH_2-$ bedeutet, und $R^7$ $C_4$–$C_{12}$-Alkyl, $C_5$–$C_8$-Cycloalkyl, $C_6$–$C_{10}$-Aryl, $C_7$–$C_{10}$-Aralkyl oder $C_7$–$C_{10}$-Alkaryl und $R^8$ Methyl oder Wasserstoff ist, und Y $C_1$–$C_{20}$-Alkyl, $C_6$–$C_{10}$-Aryl, $C_7$–$C_{10}$-Aralkyl oder $C_7$–$C_{10}$-Alkaryl ist, sowie Verbindungen der Formel VI,

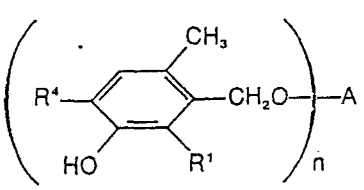

(VI)

in der $R^1$ und $R^4$ wie in Formel I definiert sind, n eine Zahl von 2–4 bedeutet und

A bei n = 2 $-C_mH_{2m}-$, worin m eine Zahl von 2 bis 12 darstellt, eine ein- oder zweimal durch –O–, –S– oder –NH– unterbrochene Gruppe $-C_mH_{2m}-$, $C_4$–$C_8$-Alkenylen, $C_4$–$C_8$-Alkinylen, Phenylen oder eine Gruppe

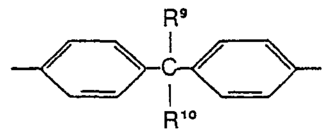

,

worin $R^9$ und $R^{10}$ unabhängig voneinander H oder $C_1$–$C_4$-Alkyl darstellen, und

A ferner bei n = 3 eine der Gruppen

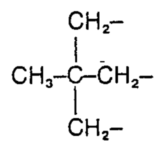

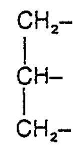

$N(CH_2CH_2-)_3-$ oder $CH_3-CH_2-C(CH_2-)_3-$ und

A bei n = 4 eine Gruppe $C(CH_2-)_4-$

bedeutet, als Stabilisatoren für organisches Material.

12. Mit den in Anspruch 11 definierten Verbindungen stabilisieren organische Polymere.

13. Stabilisierte Polymere gemäss Anspruch 12, dadurch gekennzeichnet, dass das Polymer ein Pfropfpolymer auf der Basis Acrylnitril, Butadien, Styrol (ABS) oder ein schlagfestes Polystyrol ist.

14. Mit den in Anspruch 11 definierten Verbindungen stabilisierte Schmiermittel.

**Claims**

1. A compound of the formula I

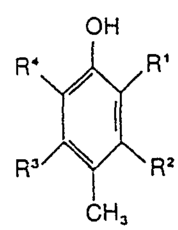

(I)

wherein $R^1$ is $C_1$–$C_4$-alkyl, $R^4$ is $C_1$–$C_{12}$-alkyl, $C_5$–$C_8$-cycloalkyl, $C_6$–$C_{10}$-aryl, $C_7$–$C_{10}$-aralkyl or $C_7$–$C_{10}$-alkaryl and $R^3$ is hydrogen or a group of the formula V, and $R^2$ is one of the radicals of the formula II, III, IV or V

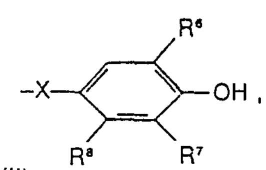

(II)

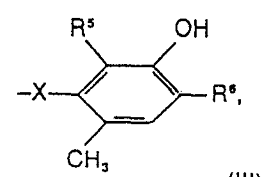

(III)

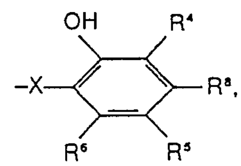

(IV)

$-CH_2OY$

(V)

wherein $R^5$ has the definition given for $R^1$ and $R^5$ can be identical to or different from $R^1$, and $R^6$ has the definition given for $R^4$ and $R^6$ can be identical to or different from $R^4$, and X is $-CH_2-$, $-CH_2OCH_2-$ or $-CH_2SCH_2-$, and $R^7$ is $C_4$–$C_{12}$-alkyl, $C_5$–$C_8$-

cycloalkyl, $C_6$–$C_{10}$-aryl, $C_7$–$C_{10}$-aralkyl or $C_7$–$C_{10}$-alkaryl and $R^8$ is methyl or hydrogen, and Y is $C_1$–$C_{20}$-alkyl, $C_6$–$C_{10}$-aryl, $C_7$–$C_{10}$-aralkyl or $C_7$–$C_{10}$-alkaryl, as well as a compound of the formula VI

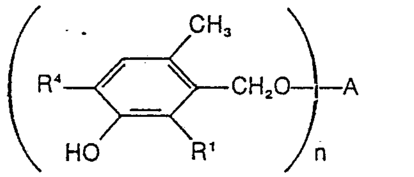
(VI)

wherein $R^1$ and $R^4$ are as defined for formula I, n is a number from 2 to 4 and, where n is 2, A is $-C_mH_{2m}$, wherein m is a number from 2 to 12, or is a $-C_mH_{2m}$-group which is interrupted once or twice by –O–, –S– or –NH–, or is $C_4$–$C_8$-alkenylene, $C_4$–$C_8$-alkynylene, phenylene or a group

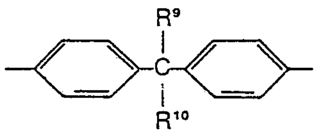
,

wherein each of $R^9$ and $R^{10}$ independently of the other is hydrogen or $C_1$–$C_4$-alkyl, and, where n is 3, A is also one of the groups

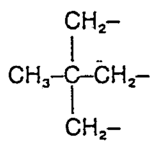
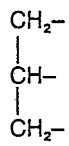

$N(CH_2CH_2-)_3-$ or $CH_3-CH_2-C(CH_2-)_3-$
and, where n is 4, A is a $C(CH_2-)_4-$ group excluding the compounds 3,3′-dihydroxy-2,2′,4,4′,6,6′-hexamethyldiphenyl-methane, 3,3′-dihydroxy-2,2′,4,4′,6,6′-hexamethyldibenzyl ether and 3-hydroxy-2,4,6-trimethylbenzyl ethyl ether.

2. A compound according to claim 1 of the formula I.

3. A compound according to claim 1 of the formula VI.

4. A compound according to claim 1 of the formula I, wherein $R^3$ is hydrogen.

5. A compound according to claim 1 of the formula I, wherein $R^2$ is a group of the formula II, III or V.

6. A compound according to claim 1 of the formula I, wherein $R^2$ is a group of the formula II or III.

7. A compound according to claim 1 of the formula I, wherein X is $-CH_2-$.

8. A compound according to claim 1 of the formula I, wherein, in formula I, $R^1$ and $R^5$ are $CH_3$, $R^4$ and $R^6$ are $C_1$–$C_4$-alkyl, and $R^7$ is t-butyl, and $R^8$ is hydrogen and Y is $C_6$–$C_{18}$-alkyl.

9. A compound according to claim 1 of the formula I, wherein $R^1$ and $R^4$ are methyl and $R^3$ is hydrogen, and $R^2$ is a group of the formula III, wherein $R^5$ and $R^6$ are $C_1$–$C_4$-alkyl.

10. A compound according to claim 1 of the formula VI, wherein $R^1$ is $CH_3$, $R^4$ is alkyl, n is 2 and A is $-C_mH_{2m}-$, wherein m is a number from 2 to 8, or is the group

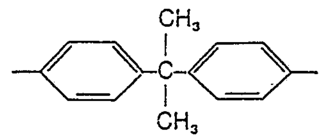

11. The use of a compound of the formula IA,

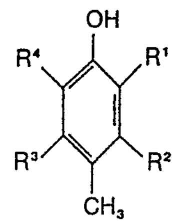
(IA)

wherein $R^1$ is $C_1$–$C_4$-alkyl, $R^4$ is $C_1$–$C_{12}$-alkyl, $C_5$–$C_8$-cycloalkyl, $C_6$–$C_{10}$-aryl, $C_7$–$C_{10}$-aralkyl or $C_7$–$C_{10}$-alkaryl and $R^3$ is hydrogen or a group of the formula V and $R^2$ is one of the radicals of the formula II, III, IV or V

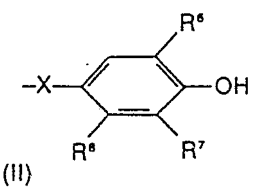
,
(II)

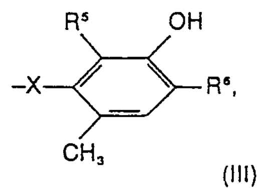
,
(III)

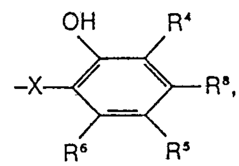
,
(IV)

$-CH_2OY$
(V)

wherein $R_5$ has the definition given for $R^1$ and $R^5$ can be identical to or different from $R^1$, and $R^6$ has the definition given for $R^4$ and $R^6$ can be identical to or different from $R^4$, and X is $-CH_2-$, $-CH_2OCH_2-$ or $-CH_2SCH_2-$, and $R^7$ is $C_4$–$C_{12}$-alkyl, $C_5$–$C_8$-cycloalkyl, $C_6$–$C_{10}$-aryl, $C_7$–$C_{10}$-aralkyl or $C_7$–$C_{10}$-alkaryl and $R^2$ is methyl or hydrogen, and Y is $C_1$–$C_{20}$-alkyl, $C_6$–$C_{10}$-aryl, $C_7$–$C_{10}$-aralkyl or $C_7$–$C_{10}$-alkaryl, as well as a compound of the formula VI

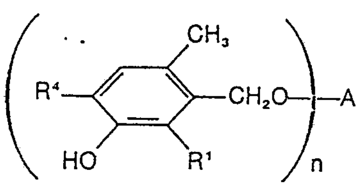
(VI)

wherein $R^1$ and $R^4$ are as defined for formula I, n is a number from 2 to 4 and, where n is 2, A is $-C_mH_{2m}$, wherein m is a number from 2 to 12, or is a $-C_mH_{2m}$-group which is interrupted once or twice by –O–, –S– or –NH–, or is $C_4$–$C_8$-alkenylene, $C_4$–$C_8$-alkynylene, phenylene or a group

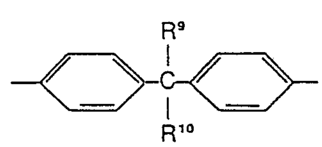

wherein each of $R^9$ and $R^{10}$ independently of the other is hydrogen or $C_1$–$C_4$-alkyl, and, where n is 3, A is also one of the groups

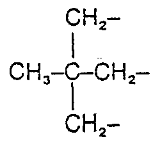 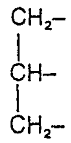

$N(CH_2CH_2-)_3-$ or $CH_3-CH_2-C(CH_2-)_3-$ and, where n is 4, A is a $C(CH_2-)_4-$ group, as a stabilizer for organic material.

12. An organic polymer stabilized with a compound defined in claim 11.

13. A stabilized polymer according to claim 12, which polymer is a graft polymer based on acrylonitrile, butadiene, styrene (ABS) or an impact-resistant polystyrene.

14. A lubricant stabilized with a compound defined in claim 11.

**Revendications**

1. Composés répondant à la formule I:

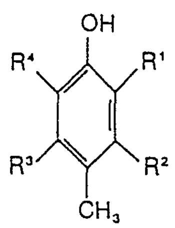 (I)

dans laquelle

$R^1$ représente un alkyle en $C_1$–$C_4$,

$R^4$ représente un alkyle en $C_1$–$C_{12}$, un cycloalkyle en $C_5$–$C_8$, un aryle en $C_6$–$C_{10}$, un aralkyle en $C_7$–$C_{10}$ ou un alkylaryle en $C_7$–$C_{10}$,

$R^3$ représente l'hydrogène ou un radical de formule V et

$R^2$ représente un radical répondant à l'une des formules II, III, IV et V:

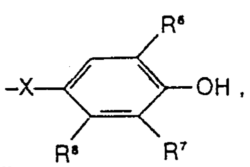 (II) 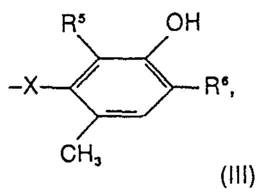 (III)

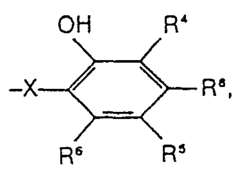 (IV) $-CH_2OY$ (V)

dans lesquelles

$R^5$ a l'une des significations qui viennent d'être données pour $R^1$ et représente ou non le même radical que $R^1$,

$R^6$ a l'une des significations qui viennent d'être données pour $R^4$ et représente ou non le même radical que $R^4$,

X représente un radical $-CH_2-$, $-CH_2OCH_2-$ ou $-CH_2SCH_2-$,

$R^7$ représente un alkyle en $C_4$–$C_{12}$, un cycloalkyle en $C_5$–$C_8$, un aryle en $C_6$–$C_{10}$, un aralkyle en $C_7$–$C_{10}$ ou un alkylaryle en $C_7$–$C_{10}$,

$R^8$ représente un radical méthyle ou l'hydrogène et

Y représente un alkyle en $C_1$–$C_{20}$, un aryle en $C_6$–$C_{10}$, un aralkyle en $C_7$–$C_{10}$ ou un alkylaryle en $C_7$–$C_{10}$,

ainsi que les composés qui répondent à la formule VI:

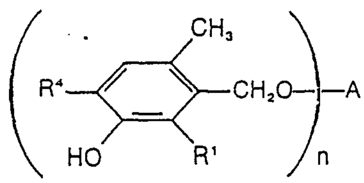 (VI)

dans laquelle

$R^1$ et $R^4$ ont les mêmes significations que dans la formule I,

n désigne un nombre de 2 à 4 et

A représente:

– lorsque n est égal à 2 un radical $-C_mH_{2m}-$ dans lequel l'indice m désigne un nombre de 2 à 12, un radical $-C_mH_{2m}-$ interrompu une ou deux fois par –O–, –S– ou –NH–, un alcénylène en $C_4$–$C_8$, un alcynylène en $C_4$–$C_8$, un phénylène ou un radical de formule:

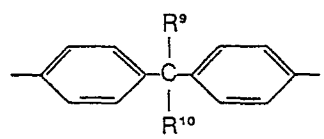

dans lequel $R^9$ et $R^{10}$ représentent chacun, indépendamment l'un de l'autre, H ou un alkyle en $C_1$–$C_4$,

– lorsque n est égal à 3 l'un des radicaux suivants:

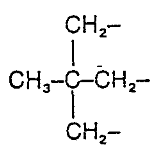 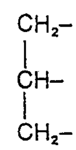

$N(CH_2CH_2-)_3-$ et $CH_3-CH_2-C(CH_2-)_3-$ et

– lorsque n est égal à 4 un radical $C(CH_2-)_4-$, sauf le dihydroxy-3,3' hexaméthyl-2,2',4,4',6,6' diphényl-méthane, l'oxyde de bis-(hydroxy-3 triméthyl-2,4,6 benzyle) et l'oxyde d'éthyle et d'hydroxy-3 triméthyl-2,4–6 benzyle.

2. Composés selon la revendication 1 qui répondent à la formule I.

3. Composés selon la revendication 1 qui répondent à la formule IV.

4. Composés selon la revendication 1 qui répondent à la formule I dans laquelle $R^3$ représente l'hydrogène.

5. Composés selon la revendication 1 qui répondent à la formule I dans laquelle $R^2$ représente un radical répondant à l'une des formules II, III et V.

6. Composés selon la revendication 1 qui répondent à la formule I dans laquelle $R^2$ représente un radical de formule II ou de formule III.

7. Composés selon la revendication 1 qui répondent à la formule I dans laquelle X représente $-CH_2-$.

8. Composés selon la revendication 1 qui répondent à la formule I dans laquelle $R^1$ et $R^5$ représentent chacun un radical $CH_3$, $R^4$ et $R^6$ représentent chacun un alkyle en $C_1$–$C_4$, $R^7$ représente un radical tert-butyle, $R^8$ l'hydrogène et Y un alkyle en $C_6$–$C_{18}$.

9. Composés selon la revendication 1 qui répondent à la formule I dans laquelle $R^1$ et $R^4$ représentent chacun un méthyle, $R^3$ représente l'hydrogène et $R^2$ représente un radical de formule III dans lequel $R^5$ et $R^6$ désignent chacun un alkyle en $C_1$–$C_4$.

10. Composés selon la revendication 1 qui répondent à la formule VI dans laquelle $R^1$ représente $CH_3$, $R^4$ un alkyle, n le nombre 2 et A un radical $-C_mH_{2m}-$ dans lequel m désigne un nombre de 2 à 8, ou A représente le radical suivant:

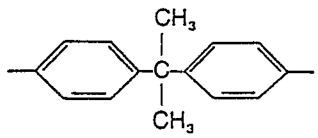

11. Application, comme stabilisants pour matières organiques, d'un composé répondant à la formule IA:

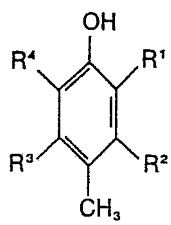
(IA)

dans laquelle

$R^1$ représente un alkyle en $C_1$–$C_4$,

$R^4$ représente un alkyle en $C_1$–$C_{12}$, un cycloalkyle en $C_5$–$C_8$, un aryle en $C_6$–$C_{10}$, un aralkyle en $C_7$–$C_{10}$ ou un alkylaryle en $C_7$–$C_{10}$,

$R^3$ représente l'hydrogène ou un radical de formule V et

$R^2$ représente un radical répondant à l'une des formules II, III, IV et V:

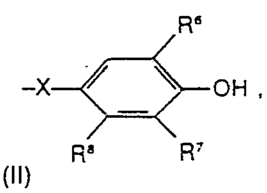
(II)

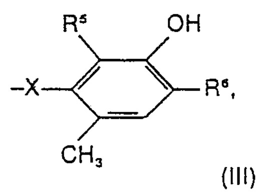
(III)

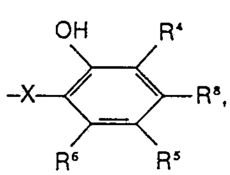
(IV)

$-CH_2OY$

(V)

dans lesquelles

$R^5$ a l'une des significations qui viennent d'être données pour $R^1$ et représente ou non le même radical que $R^1$,

$R^6$ a l'une des significations qui viennent d'être données pour $R^4$ et représente ou non le même radical que $R^4$,

X représente un radical $-CH_2-$, $-CH_2OCH_2-$ ou $-CH_2SCH_2-$,

$R_7$ représente un alkyle en $C_4$–$C_{12}$, un cycloalkyle en $C_5$–$C_8$, un aryle en $C_6$–$C_{10}$, un aralkyle en $C_7$–$C_{10}$ ou un alkylaryle en $C_7$–$C_{10}$,

$R^8$ représente un radical méthyle ou l'hydrogène et

Y représente un alkyle en $C_1$–$C_{20}$, un aryle en $C_6$–$C_{10}$, un aralkyle en $C_7$–$C_{10}$ ou un alkylaryle en $C_7$–$C_{10}$,

ainsi que les composés qui répondent à la formule VI:

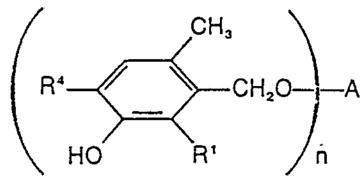
(VI)

dans laquelle

$R^1$ et $R^4$ ont les mêmes significations que dans la formule I,

n désigne un nombre de 2 à 4 et

A représente:

– lorsque n est égal à 2 un radical $-C_mH_{2m}-$ dans lequel l'indice m désigne un nombre de 2 à 12, un radical $-C_mH_{2m}-$ interrompu une ou deux fois par –O–, –S– ou –NH–, un alcénylène en $C_4$-$C_8$, un alcynylène en $C_4$-$C_8$, un phénylèné ou un radical de formule:

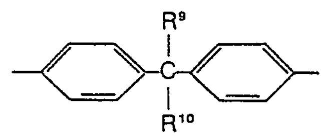

dans lequel $R^9$ et $R^{10}$ représentent chacun, indépendamment l'un de l'autre, H ou un alkyle en $C_1$–$C_4$,

– lorsque n est égal à 3 l'un des radicaux suivants:

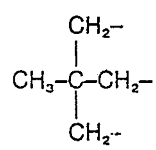

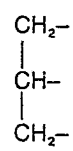

$N(CH_2CH_2-)_3-$ et $CH_3-CH_2-C(CH_2-)_3-$

et

– lorsque n est égal à 4 un radical $C(CH_2-)_4-$.

12. Polymères organiques qui ont été stabilisés au moyen des composés définis à la revendication 11.

13. Polymères stabilisés selon la revendication 12, caractérisés en ce que le polymère est un polymère greffé à base d'acrylonitrile, de butadiène et de styrène (ABS) ou un polystyrène de haute résilience.

14. Lubrifiants qui ont été stabilisés au moyen des composés qui ont été définis à la revendication 11.